# EUROPEAN PATENT APPLICATION

(11) **EP 4 268 585 A1**
(43) Date of publication of application: **01.11.2023**
(21) Application number: 21911176.2
(22) Date of filing: 12.08.2021
(51) Int. Cl.: A01H 6/46, A01H 5/10, A01H 1/04, A01H 1/02, A01H 1/06, A23L 7/10, C12Q 1/6895

(54) **FLOURY ENDOSPERM-RETAINING 'SAMKWANG'-DERIVED VARIANT RICE LINEAGE 'SAMKWANG (SA)-FLO3'**

(30) Priority: 22.12.2020 KR 20200181310
(71) Applicant: Republic of Korea (Management: Rural Development Administration), Deokjin-gu Jeonju-si, Jeollabuk-do 54875 (KR)
(72) Inventor: HA, Su Kyung, Jeollabuk-do 55365 (KR); MO, Youngjun, Jeollabuk-do 55365 (KR); JEUNG, Ji Ung, Jeollabuk-do 54955 (KR); JEONG, Jong Min, Jeollabuk-do 54865 (KR); LEE, Dong Kyu, Jeollabuk-do 54860 (KR); KIM, Jinhee, Jeollabuk-do 54072 (KR)
(74) Representative: Studio Torta S.p.A.
(86) International application number: PCT/KR2021/010746
(87) International publication number: WO 2022/139106

(57) **Abstract**

The present invention relates to novel 'Samkwang(SA)-flo3', a mutant line derived from 'Samkwang' with floury endosperm, and a use thereof. More specifically, the present invention includes 'Samkwang(SA)-flo3' with floury endosperm suitable for exclusive production of dry-milled rice flour and relates to a method for breeding the rice cultivar, a composition, a kit, a method for determining floury endosperm characteristics of rice in the next generation of the rice cultivar, and a food composition comprising the rice cultivar, seeds thereof or extracts thereof as an active ingredient.

The 'Samkwang (SA)-flo3' provided by the present invention has floury endosperm characteristics and is a mutant line similar in agricultural characteristics to the original cultivar 'Samkwang' and thus can be advantageously utilized as a novel material for various rice processed foods due to its high dry milling efficiency.

## Description

### [Technical Field]

The present invention relates to novel 'Samkwang(SA)-flo3', a mutant line derived from 'Samkwang' with floury endosperm, and a use thereof. More specifically, the present invention includes 'Samkwang(SA)-flo3' with floury endosperm suitable for exclusive production of dry-milled rice flour and relates to a method for breeding the rice cultivar, a composition, a kit, and a method for determining floury endosperm characteristics of rice in the next generation of the rice cultivar, and a food composition comprising the rice cultivar, seeds thereof or extracts thereof as an active ingredient.

### [Background Art]

Rice is traditionally the staple food in Korea, but due to changes in consumption conditions, the demand for cooked rice has been continuously declining since the 1980s. Therefore, in order to increase the competitiveness of domestic rice and expand consumption, it is important to develop rice cultivars for various uses and related processing technologies.

In order to promote high added-value in the rice processing industry, rice flour, like wheat flour, must be distributed in large marts and department stores, and various grades of rice flour must be supplied according to quality standards for each processed product group. However, due to the hardness of rice grains (grain hardness), the most commonly used rice milling method in Korea is wet milling via a waterlogging process in which rice is soaked in water and pulverized. However, wet milling has a problem of causing water pollution by generating about 500 liters of rice water to produce 100 kg of rice flour. In addition, in order to distribute rice flour, additional processes such as sterilization, drying, *etc.*, are required. Therefore, in order to expand consumption through the vitalization of the rice processing industry, the necessity of developing exclusive rice flour products suitable for dry milling has been continuously suggested.

Accordingly, the Rural Development Administration has developed 'Suweon 542', which enables the production of high-quality rice flour only by dry milling using a wheat mill due to low grain hardness of floury endosperm, among the 'Namil' mutant lines mutated by sodium azide (NaN₃) (Korean Patent No. 10-1190642, Date of Patent Grant: 2012.10.08.). Through follow-up studies, it was revealed that the floury endosperm characteristics of 'Suweon 542' were dominated by the recessive single gene *'flo7*' located on chromosome 5. In addition, by comparing the nucleotide sequences of 'Namil' and 'Suweon 542' corresponding to '*cyOsPPDK*' (cytosolic pyruvate orthophosphate dikinase protein), which is presumed to be a gene that characterizes floury endosperm, G(Namil)→A (Suweon 542) SNP in exon 8 was confirmed (Heng Wang et al. MGG, 2018 293: 1151-1158), and further, a marker that can identify the corresponding SNP was developed (Korean Application Publication No. 10-2017-0149236, Filing Date: 2017.11.10.), and an application was filed for 'novel gene with floury endosperm (International Application/KR2018/013661, Filing Date: 2018.11.09).

In addition, the Rural Development Administration has developed novel rice cultivars 'Garumi 1' and 'Garumi 2' that have reduced viviparous germination, excellent disease resistance, and excellent adaptability to late-season culture, while containing floury endosperm suitable for rice flour production using dry milling.

### [Disclosure]

### [Technical Problem]

The present invention was completed by developing 'Samkwang(SA)-flo3', which can easily be milled by a wheat mill due to low grain hardness of floury endosperm, among the fixed lines (a total of about 5,000) mutated by sodium azide from 'Samkwang', the highest quality of rice.

### [Technical Solution]

One object of the present invention provides a 'Samkwang(SA)-flo3' rice cultivar, a mutant line derived from 'Samkwang' with floury endosperm characteristics, deposited under accession number KACC 98101P.

Another object of the present invention provides a method for breeding 'Samkwang(SA)-flo3', a mutant line for 'Samkwang' rice cultivar.

Still another object of the present invention provides a composition for determining floury endosperm characteristics of rice in the next generation of 'Samkwang(SA)-flo3', including an agent capable of detecting a single nucleotide polymorphism (SNP) marker at a position corresponding to 19,721,940 bp of chromosome 5 in the standard genome (IRGSP 1.0) of rice.

Still another object of the present invention provides a kit for determining floury endosperm characteristics of rice in the next generation of 'Samkwang(SA)-flo3', including the composition.

Still another object of the present invention provides a method for determining floury endosperm characteristics of rice in the next generation of 'Samkwang(SA)-flo3', including: detecting a SNP marker at a position corresponding to 19,725,941 bp of chromosome 5 in the standard genome (IRGSP 1.0) of rice.

Still another object of the present invention provides a food composition including the rice cultivar, seeds thereof or extracts thereof, as an active ingredient.

### [Advantageous Effects]

The 'Samkwang(SA)-flo3' provided by the present invention has floury endosperm characteristics and is a mutant line similar in agricultural characteristics to the original cultivar 'Samkwang' and thus can be advantageously utilized as a novel material for various rice processed foods due to its high dry milling efficiency.

### [Brief Description of Drawings]

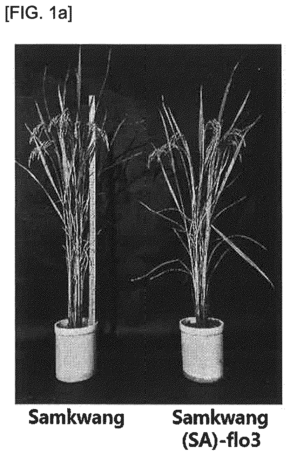

FIG. 1a is a photograph showing the novel cultivar 'Samkwang(SA)-flo3' provided by the present invention and the original cultivar 'Samkwang' that have reached the ripening stage by propagation in the experimental field of the crop breeding department of the National Institute of Crop Science in 2020 (Wanju-gun, Jeonbuk).

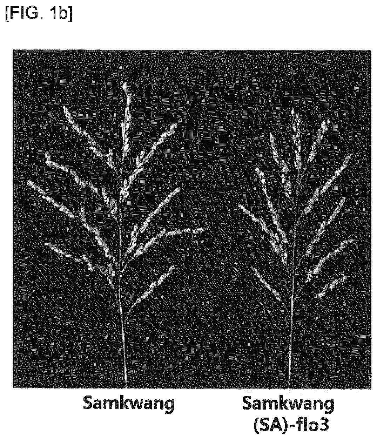

FIG. 1b is a photograph showing ears of the novel cultivar 'Samkwang (SA)-flo3' provided by the present invention and the original cultivar 'Samkwang'.

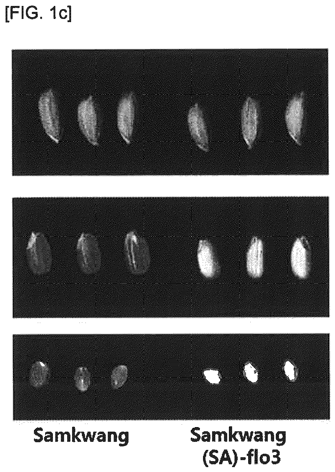

FIG. 1c is a photograph showing the appearance of hulled rice and brown rice of the novel cultivar provided by the present invention. In order to present floury endosperm, brown rice and its cross-section endosperm were shown.

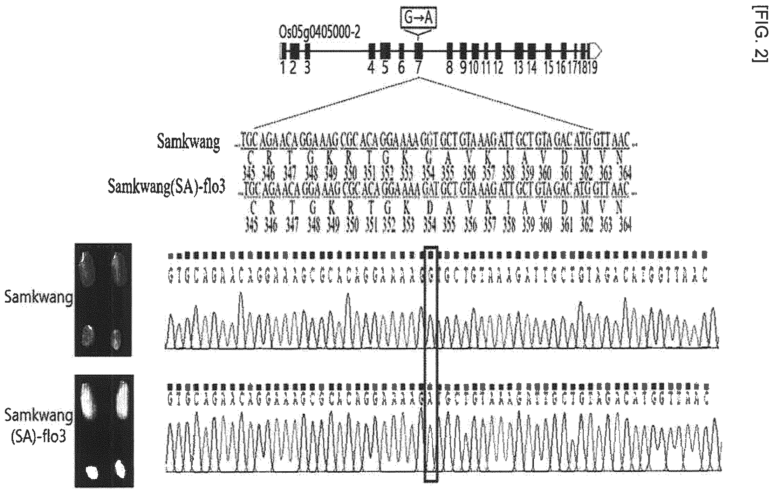

FIG. 2 is the result of determining the nucleotide sequences at the [G/A] SNP position (19,721,943 bp) between 'Samkwang' and 'Samkwang (SA)-flo3' that were confirmed in exon 7 of 'Os05g0405000-02', which is an ORF (Open Reading Frame) containing '*cyOsPPDK*' (cytosolic pyruvate orthophosphate dikinase protein) presumed to be a gene that determines floury endosperm. It was confirmed that 'Samkwang', the original cultivar and non-glutinous rice, had [G] base, and 'Samkwang(SA)-flo3' provided by the present invention had [A] base.

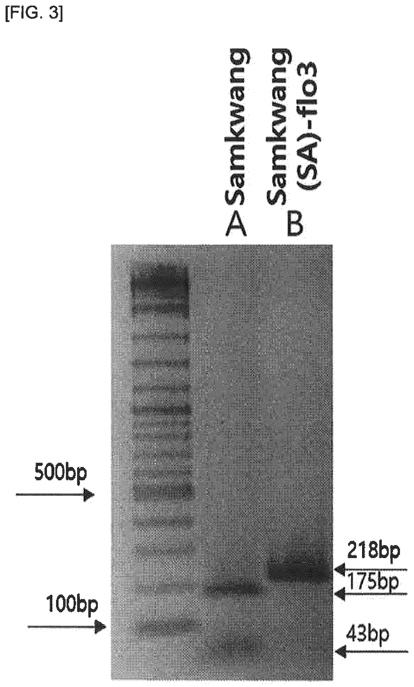

FIG. 3 is a diagram showing the nucleotide sequences and restriction enzyme (Rsal) digestion site of the fragment amplified by dCAPS primer set according to the present invention.

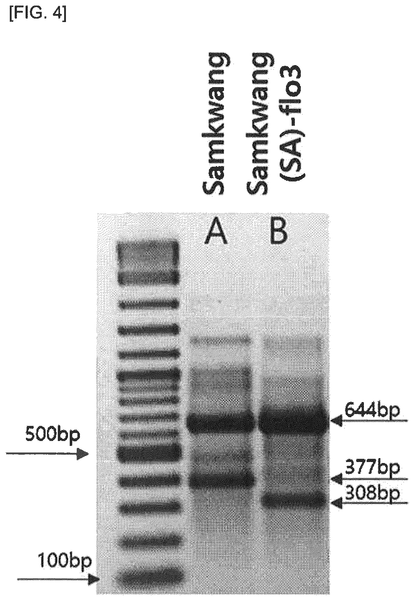

FIG. 4 shows the results of electrophoresis of 'Samkwang' and 'Samkwang(SA)-flo3', as a method using tetra-primer ARMS primer set according to the present invention,

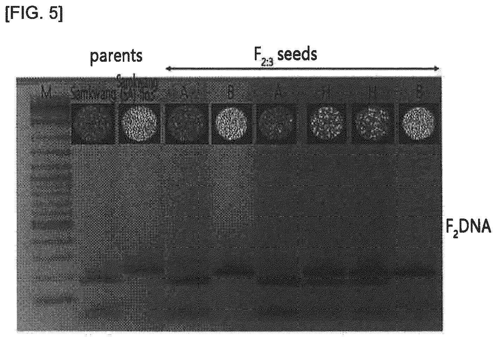

FIG. 5 shows the results of electrophoresis of hybridization progeny between 'Samkwang' and 'Samkwang(SA)-flo3', as a method using dCAPS primer set and restriction enzyme (*Rsal*) according to the present invention. Specifically, as a result of the phenotype of F_{2:3} seeds and the genotype of F2, homozygous for the allele of 'Samkwang' was marked 'A', and homozygous for the allele of 'Samkwang(SA)-flo3' was marked 'B'.

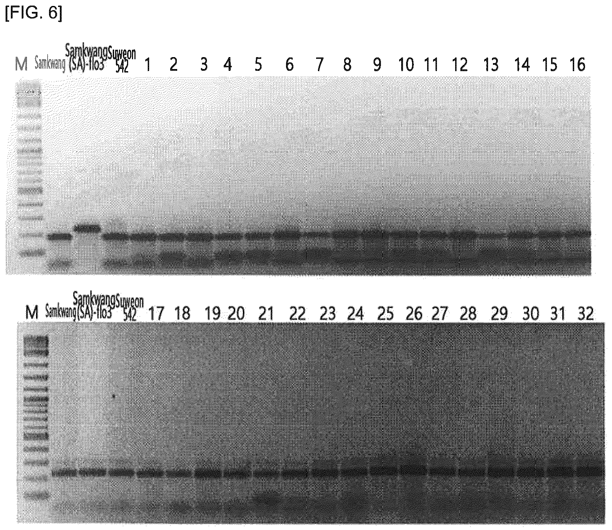

FIG. 6 shows the results of electrophoresis of domestic rice cultivars with similar genetic backgrounds, as a method using dCAPS primer set and restriction enzyme (Rsal) according to the present invention (1. Goun, 2. Ungwang, 3. Josaengheukchal, 4. Odae 1, 5. Shinunbong 1, 6. Manna, 7. Geumo 3, 8. Hwangeumbora, 9. Nunkeunheukchal, 10. Asemi, 11. Ondami, 12. Jungmo 1032, 13. Jinok, 14. Baekilmi, 15. Asemi 1, 16. Haedam Rice, 17. Mihyangbyeo, 18. Shindongjin, 19. Sujin, 20. Hojin, 21. Heukhyang, 22. Junam, 23. Goami, 24. Dongjin 1, 25. Hyangmibyeo 1, 26. Nokyang, 27. SegaeJinmi, 28. Hanareum 3, 29. Hanareum 4, 30. Geumgang 1, 31. Mokwoo, 32. Mokyang).

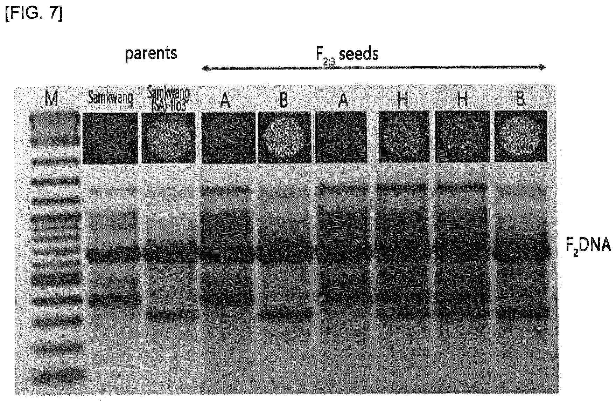

FIG. 7 shows the results of electrophoresis of hybridization progeny between 'Samkwang' and 'Samkwang(SA)-flo3', as a method using tetra-primer ARMS primer set according to the present invention. Specifically, as a result of the phenotype of F_{2:3} seeds and the genotype of F2, homozygous for the allele of 'Samkwang' was marked 'A', and homozygous for the allele of 'Samkwang(SA)-flo3' was marked 'B'.

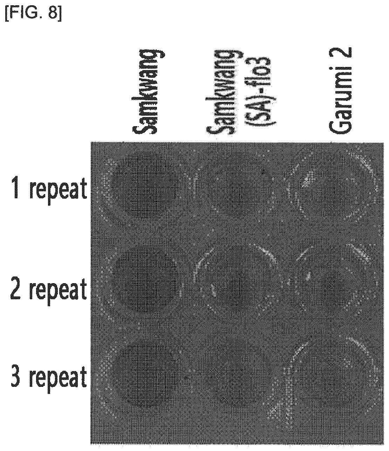

FIG. 8 is a photograph of samples treated with a commercial kit (Starch Damage Assay Kit, Megazyme) to measure the damaged starch content of rice flour produced by a test wheat mill and colored to quantify the damaged starch content with a spectrophotometer. All rice flour produced from the 6-fraction series (B1, R1, B2, R2, B3, R3) of 'Buhler MLU-02', a test wheat mill, was mixed and used. The average measured damaged starch content was 12.0% for 'Samkwang', 6.0% for 'Samkwang(SA)-flo3' and 4.9% for 'Garumi 2'.

### [Detailed Description of Preferred Embodiments]

Hereinafter, the present invention will be described in detail. Meanwhile, each description and embodiment disclosed herein can be applied to other descriptions and embodiments, respectively. That is, all combinations of various elements disclosed herein fall within the scope of the present invention. Further, the scope of the present invention is not limited by the specific description described below.

Additionally, those of ordinary skill in the art may be able to recognize or confirm, using only conventional experimentation, many equivalents to the particular aspects of the invention described herein. Furthermore, it is also intended that these equivalents be included in the present invention.

One aspect of the present invention provides a 'Samkwang(SA)-flo3' rice cultivar, a mutant line derived from 'Samkwang' with floury endosperm characteristics, deposited under accession number KACC 98101P.

As used herein, the term "floury endosperm" refers to a mutant having the property of having a cloudy and opaque appearance similar to that of glutinous rice and having a loose arrangement of starch granules. Dry and wet millings are utilized for the production of rice flour. Dry milling is a simple process, but there is a problem that the damaged starch increases, while wet milling improves the processability, but the time and cost required for soaking and drying are a limiting factor. In order to contribute to the vitalization of the rice processing industry, it is required to diversify processing cultivars having characteristics suitable for each use and to develop cultivars excellent in milling quality, and such floury endosperm characteristics are one of the important characteristics that can overcome the problems of conventional dry milling.

Rice containing floury endosperm characteristics is advantageous for dry milling, and dry milling can produce rice flour with minimal water pollution, and thus is environmentally friendly and can reduce processing costs.

The rice cultivar according to the present invention is a cultivar selected as a floury endosperm from the 'Samkwang' mutant fixed lines by treatment with sodium azide (NaN₃) via visual rating, and the cultivar was named 'Samkwang(SA)-flo3' and deposited at the National Institute of Agricultural Sciences, Rural Development and Administration on October, 29, 2020, with Accession No. KACC 98101P.

The 'Samkwang (SA)-flo3' rice cultivar was transferred from domestic deposit at the Korean Agricultural Culture Collection in the National Institute of Agricultural Sciences, Rural Development and Administration to international deposit under the Budapest on July 5, 2021, with International Accession No. KACC 88004BP.

The rice cultivar may have the following characteristics:
(a) Heading date: August 14^{th} ±10 days
(b) Culm length (cm): 76±20
(c) Panicle length (cm): 19±10
(d) Tiller number (number): 15±5
(e) 1000-grain weight of brown rice (g): 18.5±5.0
(f) Length of brown rice (mm): 5.09±3.00
(g) Width of brown rice (mm): 2.97±2.00
(h) Length to width ratio of brown rice: 1.72±0.50.
(g) Genotype of floury endosperm gene (*flo4-6*) of 'Samkwang(SA)-flo3'
(h) Sequence of SNP present in exon 7 of ORF of *cyOsPPDK* (cytosolic pyruvate orthophosphate dikinase protein) gene, a floury endosperm gene, is A
(i) Grain hardness of seeds weighing 3.0±0.34 kg
(j) Heading date of normal season culture on August 14 until finish
(k) Average size of seed powder of 65.3±0.86 µm
(l) Damaged starch ratio of seed powder of 6.0%.

Another aspect of the present invention provides a method for breeding 'Samkwang(SA)-flo3', a mutant line of progeny for the 'Samkwang' rice cultivar.

The terms used herein are as described above.

The method may include:
(a) immersing 'Samkwang' seeds in a sodium azide (NaN₃) solution, and advancing M2 seeds harvested from plants (M1) grown by germination thereof to M7 generation based on a line breeding method, thereby establishing fixed mutant lines; and
(b) selecting a rice cultivar with floury endosperm among the seeds of each established line.

The step (a) may be carried out by immersing 'Samkwang' seeds in a sodium azide (NaN₃) solution, and advancing M2 seeds harvested from plants (M1) grown by germination thereof to M7 generation based on a line breeding method, thereby establishing fixed mutant lines.

The concentration of the sodium azide solution in step (a) is not particularly limited, but as an example, it may be 10 mM to 200 mM, and as another example, it may be 50 mM to 150 mM, and as still another example, it may be 100 mM.

The immersion temperature in step (a) is not particularly limited, but as an example, it may be 15°C to 30°C, and as another example, it may be 25°C.

The immersion time of step (a) is not particularly limited, but as an example, it may be 1 to 12 hours, as another example, it may be 3 to 9 hours, and as still another example, it may be 6 hours.

The step (b) may be carried out by selecting a rice cultivar with floury endosperm among the seeds of each established line.

In an example, in step (b), each seed of the mutant lines established in step (a) was reproduced, and a line expressing endosperm similar to 'Suweon 542 (floury)' was visually selected.

In the present invention, the steps (a) and (b) may be performed with appropriate changes according to changes in the characteristics of floury endosperm, mutant lines, *etc.*

Still another aspect of the present invention provides a composition for determining floury endosperm characteristics of the line of progeny for 'Samkwang(SA)-flo3' rice, including an agent capable of detecting a single nucleotide polymorphism (SNP) marker at a position corresponding to 19,721,940 bp of chromosome 5 in the standard genome (IRGSP 1.0) of rice.

The terms used herein are as described above.

As used herein, the term "polymorphism" may occur when two or more alleles exist in one locus, and the term "single nucleotide polymorphism (SNP)" may occur when a single nucleotide differs among the polymorphic sites. As used herein, the term "allele" refers to several forms of a gene exists on the same locus of homologous chromosomes, and the SNP has two types of alleles (biallele).

In the present invention, the SNP is present at position 3,403 (19,721,940 bp) of the polynucleotide sequence (ch;19,718,538∼19,726,410) derived from 'Samkwang(SA)-flo3' represented by SEQ ID NO: 1 located on chromosome 5. More specifically, G at position 3,403 is substituted with A, and accordingly, it may refer to a mutation of glycine (Gly, G), the amino acid at position 354, to aspartic acid (Asp, D).

In the present invention, the agent capable of detecting the SNP marker may be a primer capable of amplifying a polynucleotide consisting of 10 to 300 bases containing the single nucleotide polymorphic site present at position 3,403 of the polynucleotide sequence represented by SEQ ID NO: 1, or a probe that specifically hybridizes with the polynucleotide

Specifically, the agent capable of detecting the SNP marker may be a primer set consisting of primers represented by SEQ ID NO: 4 to SEQ ID NO: 7, but is not limited thereto.

As used herein, the term "primer" refers to a short nucleic acid sequence having a free 3' hydroxyl group, which is able to form a base pair with a complementary template and serves as a starting point for replication of a template strand. The appropriate length of the primer may vary according to the purpose of use, and may generally consist of 15 to 30 nucleotides. A primer sequence is not necessarily completely complementary with a template but should be complementary enough to hybridize to a template. The primer may hybridize to a DNA sequence containing a polymorphic site to amplify a DNA fragment containing the polymorphic site. For the purpose of the present invention, the primer may amplify a single nucleotide polymorphic site closely related to floury endosperm characteristics, and may be, for example, a primer set consisting of primers represented by SEQ ID NO: 4 to SEQ ID NO: 7, but is not limited thereto.

The primer set may consist of any one nucleotide sequence having preferably 80% or more sequence homology, more preferably 90% or more sequence homology, and even more preferably 95% or more sequence homology, and most preferably 99% or more sequence homology with each of the nucleotide sequences represented by SEQ ID NO: 4 to SEQ ID NO: 7, but any nucleotide sequence may be included without limitation as long as it can amplify the above-described SNP site.

The primer or probe of the present invention may be chemically synthesized using a phosphoramidite solid support method or other widely known methods. These nucleic acid sequences may also be modified using any means known in the art. Non-limiting examples of such modifications include methylation, capsulation, replacement of one or more native nucleotides with analogues thereof, and inter-nucleotide modifications, for example, modifications to uncharged conjugates (e.g., methyl phosphonate, phosphotriester, phosphoroamidate, carbamate, *etc*.) or charged conjugates (e.g., phosphorothioate, phosphorodithioate, *etc*.).

Still another aspect of the present invention provides a kit for determining floury endosperm characteristics of the line of progeny for 'Samkwang(SA)-flo3' rice, including the composition

The terms used herein are as described above.

The kit according to the present invention, in addition to the above composition, may further include a DNA polymerase, dNTP, and a PCR buffer that facilitate the PCR, and in addition, the kit may further include components necessary for electrophoresis to confirm the amplification of PCR product, or identification criteria for known cultivars.

Even another aspect of the present invention provides a method for determining floury endosperm characteristics of the line of progeny for 'Samkwang(SA)-flo3' rice, including: detecting a SNP marker at a position corresponding to 19,725,941 bp of chromosome 5 in the standard genome (IRGSP 1.0) of rice.

The terms used herein are as described above.

In the standard genome (IRGSP 1.0) of rice, the SNP marker at a position corresponding to 19,721,940 bp of chromosome 5 may have A base.

The step of detecting a SNP marker may include:
(a) performing PCR using a primer set consisting of primers represented by SEQ ID NO: 4 to SEQ ID NO: 7 based on the genomic DNA isolated from a sample as a template; and
(b) confirming the product amplified by the PCR.

Still another aspect of the present invention provides a food composition including the rice cultivar, seeds thereof or extracts thereof, as an active ingredient.

The terms used herein are as described above.

Specifically, the rice cultivar is 'Samkwang(SA)-flo3', which is as described above.

The seeds of the cultivar of the present invention can be dry milled to produce high-quality rice flour with fine particles and less damage to starch granules, and accordingly, a food composition containing the rice cultivar as an active ingredient has price competitiveness and excellent texture.

The food composition of the present invention may include rice flour produced from the seeds of the rice cultivar.

The rice flour may be produced by dry milling.

The food composition of the present invention includes all types of processed rice foods, functional foods, nutritional supplements, health functional foods, and food additives. This type of food composition can be prepared in various forms according to conventional methods known in the art.

For example, the processed rice foods (*e.g*., rice cake/noodles (rice cake, rice cake for rice cake soup, rice cake for stir-fried rice cake, noodles, ramen, raw noodles, traditional rice cake), rice snacks (biscuits, hardtack, snacks, Korean traditional sweets, fried rice, scorched rice), rice flour (raw rice flour, alpha rice flour, wet rice flour), liquors (turbid rice-wine/YAG-JJU, soju, beer, rice wine), seasoning food (taffy, fermented soybean paste, vinegar), and other rice processed products (porridge, sweet rice drink, snack additives, skewers, mixed grain powder, rice beverages, rice bread, processed cooked rice)) may be prepared by processing the rice cultivar provided in the present invention or seeds thereof.

As used herein, the term "health functional food" is the same term as a food for special health use (FOSHU), and refers to a food which is processed to effectively exert a body-regulating function in addition to nutrient supply, thus having high medicinal and medical effects. In particular, the term "functionality" refers to controlling nutrients for the structure of functions of the human body or providing useful effects for hygienic purposes, such as physiological effects, *etc.*

There is no particular limitation on other ingredients that may be included in the food composition of the present invention other than the 'Samkwang(SA)-flo3' rice cultivar, seed thereof or extracts thereof as an essential ingredient, and various herbal extracts, food supplement additives, or natural carbohydrates may be contained as additional components with conventional foods. In addition, the food supplement additives include conventional food supplement additives in the art, for example, flavoring agents, flavors, coloring agents, fillers, stabilizers, *etc*.

The food of the present invention may be prepared according to a method commonly employed in the art, and raw materials and ingredients commonly used in the art may be added when preparing the food. Additionally, the formulation of the food is not particularly limited so long as it is recognized as a formulation of the food or food. The food composition of the present invention may be prepared in various formulations, and the food composition of the present invention uses a food as a raw material unlike generic drugs, and thus has no side effects that may occur during long-term administration thereof and is highly portable.

The content of plants, seeds thereof or extracts thereof that may be included in the food composition of the present invention may be, for example, 0.01% to 95% by weight based on the finally prepared food, but is not limited thereto.

### [Mode for Carrying Out the Invention]

Hereinafter, the present invention will be described in more detail with reference to the following Examples and Experimental Examples. However, these Examples and Experimental Examples are for illustrative purposes only and the scope of the invention is not limited by these Examples and Experimental Examples.

### Example 1: Selection of Novel Rice Cultivar with Floury Endosperm

### Example 1-1: Establishment of Fixed Line of 'Samkwang' Mutant Fixed Line

Mutant line treated with sodium azide (NaN3) diluted in potassium phosphate buffer as a mutagen to 'Samkwang', a domestic high-quality rice cultivar (*Oryza sativa L*.) was secured. One ear was harvested from M1 plants (M2 seeds), and from thereon, a fixed line was established by advancing the generations according to a line breeding method until M7 generation. In each generation, one plant with high fertility was randomly selected for each line, and the next line was developed using the ear-to-row method. In the M7 plant generation, it was determined that individuals within each line showed a uniform phenotype and were genetically fixed, and a total of about 5,000 lines were confirmed.

### Example 2: Evaluation of Aaronomic Traits

The major agronomic traits of the 'Samkwang(SA)-flo3' cultivar, such as culm length, panicle length, and tiller number, were evaluated based on normal-season culture fertilization conditions in the experimental field of the National Institute of Crop Science (NICS) (Wanju).

As shown in Table 1 and FIGS. 1a and 1b, as a result of comparing the agronomic traits of the 'Samkwang(SA)-flo3' cultivar selected in Example 1 and the original cultivar (Samkwang), it was confirmed that the 'Samkwang(SA)-flo3' cultivar had low grain hardness, spikelet number per panicle, and 1000-grain weight of brown rice than those of 'Samkwang', but maintained similar levels of agronomic traits such as heading date, culm length, panicle length, tiller number, etc., as compared to the mid-late maturing original cultivar (Samkwang).

Additionally, 'Samkwang(SA)-flo3' was judged to be suitable for the selection requirements of exclusive mid-late maturing rice flour cultivar on November 8, 2020, and was designated as 'Jeonju672' in the local adaptability trials for novel elite line of rice in 2021.

**[Table 1]**

| Major agronomic traits of 'Samkwang(SA)-flo3' and original cultivar ('Samkwang') confirmed in the preliminary yield trial (PYT) conducted under normal-season culture conditions at the National Institute of Crop Science (Wanju, Jeonbuk) in 2020. | | | | | | | | | | | |
|---|---|---|---|---|---|---|---|---|---|---|---|
| Cultivar | Transplanting date (mm/dd) | Heading date (mm/ dd) | Transplantation ~Heading (days) | Culm length (cm) | Panicle length (cm) | Tiller number (Perplant) | Spikelet number (Perpanicle) | 1000-grain weight of brown rice (g) | Brown rice recovery (%) | Brown rice yield (kg/10a) | Score |
| Samkwan 9 | 5/ 30 | 8/14 | 76 | 76 | 19 | 15 | 87 | 18.5 | 88% | 445 | 82% |
| (SA)-flo3 | | | | | | | | | | | |
| 'Samkwa ng' | 5/ 30 | 8/13 | 75 | 77 | 20 | 14 | 101 | 21.3 | 84% | 541 | 100% |

Accordingly, the 'Samkwang(SA)-flo3' was deposited at the Korean Agricultural Culture Collection in the National Institute of Agricultural Sciences, Rural Development and Administration on October 29, 2020 under Accession No. KACC 98101P.

### Example 3: Selection of Rice Cultivar with Floury Endosperm

As a result of evaluating the appearance of brown rice for the 'Samkwang' mutant fixed group confirmed in Example 1-1, a line with cloudy endosperms was selected. As shown in FIG. 1c, it was possible to distinguish between the floury endosperm and non-glutinous rice visually.

### Example 3-1: DNA Extraction and Phenotypic Evaluation of Endosperm F_{2:3} Genetic Analysis Population

For genetic analysis of floury endosperms, the F₂ population, which was grown by self-fertilization of F1 plants derived from the cross between the original variety 'Samkwang' and 'Samkwang(SA)-flo3', was published in the greenhouse of the National Institute of Crop Sciences in June 2020. As a result of the visual evaluation of brown rice of the F₂ seeds from the cross between 'Samkwang' and 'Samkwang(SA)-flo3', a 3:1 segregation ratio of 1,122 non-glutinous rice to 363 endosperms was exhibited. A total of 419 individuals (338 non-glutinous rice and 81 endosperm) was randomly selected from the developed F₂ population and confirmed as a genetic analysis group to search for the locus that dominates the flour endosperm characteristics of 'Samkwang(SA)-flo3'.

DNA was extracted by collecting a part of the leaves of the genetic analysis group, 'Samkwang(SA)-flo3' and the original variety 'Samkwang' and modifying the CTAB method (Murray and Thompson, 1980). The extracted DNA was confirmed by electrophoresis on a 1% agarose gel, and diluted to an appropriate concentration for use in PCR reaction after quantification using a NanoDrop spectrophotometer (Thermo Fisher scientific, USA).

### Example 4: Identification of Single Nucleotide Polymorphic Sites Related to Floury Endosperm Characteristics of 'Samkwang(SA)-flo3'

### Example 4-1: Extraction of DNA

DNA of 'Samkwang' and 'Samkwang(SA)-flo3' with floury endosperm characteristics were extracted by the CTAB method (Murray & Thomason, 1980) after collecting respective young leaves.

The extracted DNA was confirmed by electrophoresis on a 0.8% agarose gel and diluted to 5 ng/µL for use in PCR after quantification using a NanoDrop spectrophotometer (Thermo Fisher Scientific, USA).

### Example 4-2: Comparison of PPDK1 (OS05G045000-02) Gene Sequences of 'Samkwang' and 'Samkwang(SA)-flo3'

Based on the information of standard genome of rice (IRGSP 1.0), transcript information of the candidate gene PPDK1 (pyruvate, phosphate dikinase1) located on chromosome 5 was confirmed, and the nucleotide sequence of PPDK1 in OS05G0405000-02(19,718,538-19,737,605 bp) was analyzed (SEQ ID NO: 1, Accession No. MW349590). As shown in FIG. 2, the SNP associated with the floury endosperm characteristics, which is a position corresponding to 19,721,940 bp in the IRGSP standard genome, was found to be 'G' in 'Samkwang' and 'A' in 'Samkwang (SA)-flo3'. More specifically, it was confirmed that glycine (Gly, G) the amino acid at position 354 of OS05G0405000-02 was mutated to aspartic acid (Asp, D).

### Example 5: Preparation of Primer Set for Determination of Floury Endosperm Characteristics

### Example 5-1: Preparation of dCAPS Primer Set

For the SNPs identified in Example 4, dCAPS primers were prepared using dCAPS Finder 2.0 (http://helix.wustl.edu/dcaps/dcaps/html). For the region amplified by dCAPS, a total of 218 bp was amplified by adding 21 bp of overhang to 19,718,217,67-19,721,944 bp (197 bp) in chromosome 5 containing the SNP (19,721,940 bp) that has been determined to be polymorphic, and primers with 1 bp mismatch were searched.

**[Table 2]**

| Primer Sequences used in dCAPS Primers | | |
|---|---|---|
| Primers | Sequence Information | Note |
| Flo4-6-Rsal - F | GATTTGAAACGTCTTGATCATGC(SEQ ID NO: 2) | Forward primer |
| *Flo4-6-Rsal* - R | AGCATGAACTTGGTCACCTTCTGTCTACAGCAATCTTTAC AGTA(SEQ ID NO: 3) | Reverse primer, including over-hang (21bp) |

PCR was performed using 10 ng of DNA, 5 pmol of forward and reverse primers, 0.2 mM dNTP mix, 1X PCR buffer [50 mM KCI, 10 mM Tris-HCI (pH 9.0), 0.1% Triton X-100, 1.5 mM MgCl₂] and 1 unit of Taq polymerase for a total volume of 20 uL. PCR was performed by initial denaturation at 95°C for 5 minutes, followed by 40 cycles of 95°C for 20 seconds, 58°C for 30 seconds, and 72°C for 60 seconds, with a final extension at 72°C for 10 minutes.

As the restriction enzyme, *Rsal* that cleaves the '5...GTAC....3' site was used, and as shown in FIG. 3, since 'Samkwang' has a GTAC sequence by SNP, it had been designed to be cleaved by the restriction enzyme, and 'Samkwang(SA)-flo3' is not cleaved by the restriction enzyme. The treatment by the restriction enzyme was carried out using 1X enzyme buffer [50 mM potassium acetate, 20 mM Tris-acetate, 10 mM magnesium acetate, 100 ug/mL BSA], 10 ng of PCR product and 5U *Rsal* (NEB, UK) for a total of 15 uL.

The resultant was subjected to electrophoresis on an agarose gel, and then the genotype was determined.

As a result, as shown in Table 3 and FIG. 3, it was confirmed that although 218 bp of 'Samkwang' and 'Samkwang(SA)-flo3' were amplified, 'Samkwang' was cleaved into 175 bp and 43 bp by the *Rsal* restriction enzyme, and 'Samkwang (SA)-flo3' was not cleaved.

**[Table 3]**

| Sites Cleaved by Restriction Enzyme *Rsal* | | |
|---|---|---|
| | Samkwang(SA)-flo 3 | 'Samkwang' |
| PCR Fragment Size (bp) | 218 | 218 |
| Fragment Size after Treatment with Restriction Enzyme | 218 | 175+43 |

### Example 5-2: Preparation of Tetra-primer ARMS Primer Set

In order to prepare primers that can omit the restriction enzyme treatment step for the searched SNPs, tetra-primer ARMS-PCR primers were developed using Primer 1 (primer design for tetra-primer ARMS-PCR, http://primer1.soton.ac.uk/primer1.html). As shown in Table 4, a primer set capable of discriminating SNP of 'Samkwang (SA)-flo3' with floury endosperm characteristics without restriction enzyme treatment was selected.

DNA of 'Samkwang' and 'Samkwang (SA)-flo3' with floury endosperm characteristics was extracted using CTAB (Cetyltrimethyl ammonium bromide) method (Murray & Thomason, 1980) after collecting respective leaves.

The extracted DNA was confirmed by electrophoresis on a 0.8% agarose gel and diluted to 5 ng/µ for use in PCR after quantification using a NanoDrop spectrophotometer (Thermo Fisher Scientific, USA).

**[Table 4]**

| Primer Sequences used in Tetra Primer ARMS-PCR | | | |
|---|---|---|---|
| Primers | Sequence Information | Tm (°C) | Note |
| *F*/*o4-6*_FO | AATTTGCTTTATGCATCAATACC(SEQ ID NO: 4) | 58 | Forward outer primer |
| *Flo4-6_*RO | CTGATTTATTCTAAGCACGCAA(SEQ ID NO: 5) | 58 | Reverse outer primer |
| *Flo4-6_*FI | TGTCTACAGCAATCTTTACAGAAC(SEQ ID NO: 6) | 58 | Forward inner primer (G allele) |
| *Flo4-6_*RI | AAAGCGCACAGGAAACGA(SEQ ID NO: 7) | 58 | Reverse inner primer (A allele) |

Specifically, when PCR was performed using *Flo4-6* tetra-primer ARMS-PCR primers, it was found that 'Samkwang' with a G allele had a PCR fragment of 644 bp by *Flo4*-6-outer-F and *Flo4*-6-outer-R and a fragment of 377 bp by *Flo4*-6-inner-F(G) binding to the G allele and *Flo4*-6-outer-R, while Samkwang(SA)-flo3' with an A allele had a PCR fragment of 644 bp by *Flo4*-6-outer-F and *Flo4*-6-outer-R and a fragment of 308 bp by *Flo4*-6-inner-R(A) binding to the A allele and *Flo4*-6-outer-F.

**[Table 5]**

| Site cleaved by Tetra Primer-ARMS | | |
|---|---|---|
| | Samkwang(SA)-flo3 | 'Samkwang' |
| PCR Fragment Size | 308+644 | 377+644 |
| (bp) | | |

PCR was performed using 10 ng of DNA, 5 pmol of a total of 4 primers, 0.2 mM dNTP mix, 1X PCR buffer [50 mM KCI, 10 mM Tris-HCI (pH 9.0), 0.1% Triton X-100, 1.5 mM MgCl₂] and 1 unit of Taq polymerase for a total volume of 20 uL. PCR was performed by initial denaturation at 95°C for 5 minutes, followed by 40 cycles of 95°C for 20 seconds, 58°C for 30 seconds, and 72°C for 60 seconds, with a final extension at 72°C for 10 minutes. The resulting product was subjected to electrophoresis using a 4% agarose gel, and then the genotype was determined.

As a result, as shown in FIG. 4, a specific fragment of 'Samkwang(SA)-flo3' could be obtained by performing PCR using the prepared *Flo4-6* tetra-primer ARMS-PCR primer. In other words, it was found that the floury endosperm characteristics of 'Samkwang(SA)-flo3' could be effectively determined through these differences.

### Example 6: Verification of Effect of Primers for Determining Floury Endosperm Characteristics of 'Samkwang(SA)-flo3'

### Example 6-1: Verification of Effect of F_{2:3} dCAPS Primer Set for 'Samkwang'×'Samkwang(SA)-flo3'

419 plants of F_{2:3} from the cross between 'Samkwang' and 'Samkwang(SA)-flo3' were analyzed under the same conditions as in Example 5 using 'Samkwang' and 'Samkwang(SA)-flo3' as male and female parents. When plants cleaved into 175 bp and 43 bp by restriction enzyme *Rsal* were defined as type A and plants not cleaved by the restriction enzyme *Rsal* were defined as type B, 'Samkwang' was found to be type A, and 'Samkwang(SA)-flo3' with floury endosperm was found to be type B. As shown in FIG. 5, the genotypes of 338 plants of F_{2:3} non-glutinous rice from the cross were found to be type A and heterozygous, and the genotypes of 81 plants of F_{2:3} floury endosperm were found to be type B, confirming that the phenotype of non-glutinous rice was dominant to that of endosperm. As a result, it was found that the SNP and dCAPS primers according to the present invention can be applied to effectively determine floury endosperm characteristics.

### Example 6-2: Verification of Effect of dCAPS Primer Set for Domestic Rice Cultivars

In addition, in order to confirm the selection effect in populations with a similar genetic background, DNA of 32 domestic rice cultivars cultivated in Korea was analyzed under the same conditions as in Example 5 (1. Goun, 2. Ungwang, 3. Josaengheukchal, 4. Odae 1, 5. Shinunbong 1, 6. Manna, 7. Geumo 3, 8. Hwangeumbora, 9. Nunkeunheukchal, 10. Asemi, 11. Ondami, 12. Jungmo 1032, 13. Jinok, 14. Baekilmi, 15. Asemi 1, 16. Haedam Rice, 17. Mihyangbyeo, 18. Shindongjin, 19. Sujin, 20. Hojin, 21. Heukhyang, 22. Junam, 23. Goami, 24. Dongjin 1, 25. Hyangmibyeo 1, 26. Nokyang, 27. SegaeJinmi, 28. Hanareum 3, 29. Hanareum 4, 30. Geumgang 1, 31. Mokwoo, 32. Mokyang).

As a result, as shown in FIG. 6, only 'Samkwang (SA)-flo3' with floury endosperm characteristics was found to be type B, and all the others were found to be type A.

As a result, it was found that the SNP and dCAPS primers according to the present invention can be applied to effectively determine floury endosperm characteristics. In addition, it was confirmed that the SNP determining the floury endosperm was different and distinct from 'Suweon 542' (floury endosperm, *flo7*).

### Example 6-3: Verification of Effect of Tetra-Primer ARMS Primer Set

419 plants of F_{2:3} from the cross between 'Samkwang' and 'Samkwang(SA)-flo3' were analyzed under the same conditions as in Example 5 using 'Samkwang' and 'Samkwang(SA)-flo3' as male and female parents. When plants cleaved into 377 bp by tetra-primer ARMS PCR were defined as type A and plants cleaved into 308 bp by tetra-primer ARMS PCR were defined as type B, 'Samkwang' was found to be type A, and 'Samkwang(SA)-flo3' with floury endosperm was found to be type B. As shown in FIG. 7, the genotypes of 338 plants of F_{2:3} non-glutinous rice from the cross were found to be type A and heterozygous, and the genotypes of 81 plants of F_{2:3} floury endosperm were found to be type B, confirming that the phenotype of non-glutinous rice was dominant to that of endosperm. As a result, it was found that the SNP and tetra-primer ARMS primers according to the present invention can be applied to effectively determine floury endosperm characteristics.

### Example 7: Analysis of Physicochemical Properties of Rice Flour

### Example 7-1: Analysis of Grain Hardness

In order to compare whether the floury endosperm characteristics, which easily breaks when pressure is applied due to its low grain hardness, were well expressed in 'Samkwang(SA)-flo3' with the control group, TA.XTplus texture analyzer (Stable Micro Systems Ltds. UK) was used to measure grain hardness. In particular, pressure was applied to the brown rice using a probe having a diameter of 5 mm (text speed; 0.4 mm/sec, trigger force; 40.0 g) to measure the pressure at the grain breakage point, which was repeated 50 times for each sample, and the mean and standard deviation were calculated.

As a result, as shown in Table 6 below, it was confirmed that the grain hardness of 'Samkwang(SA)-flo3' (3.0 kg) was very low in non-glutinous rice such as the original cultivar 'Samkwang' (9.2 kg), and was lower than that of 'Garumi 2' (3.1 kg), which is an existing cultivar for dry-milled flour production.

**[Table 6]**

| Grain Hardness and Physicochemical Properties of Rice Flour Produced by Test Mill | | | | | |
|---|---|---|---|---|---|
| Variety | Grain hardness (kg) n=50 | Physicochemical properties of Rice Flour Produced by Test Mill 'Buhler MLU-202' | | | |
| | | Mean particle size (µm) | Damaged starch (%) | Lightness (CIE value) | Ashe (%) |
| Samkwang (SA)-flo3 | 3.0±0.34 | 65.3±0.86 | 6.0±0.09 | 92.6±1.82 | 0.64±0.01 |
| 'Samkwang | 9.2±0.82 | 91.1±0.73 | 12.0±0.13 | 93.2±1.49 | 0.67±0.01 |
| | | | | | |
| 'Garumi 2' | 3.1±0.33 | 61.5±1.36 | 4.9±0.02 | 91.2±0.03 | 0.58±0.01 |

### Example 7-2: Production of Rice Flour using Dry Mill

Brown rice was used for all rice samples in consideration of the properties of wheat that the seed coats are dehulled (naked hull). The 'Samkwang(SA)-flo3', 'Samkwang' (original cultivar) and control group (Garumi 2) were dry milled under the same conditions using 'Buhler MLU-202' laboratory mill (Buhler Bros. Inc. Swiss). For reference, the Buhler mill consists of three sets of break rolls (B1, B2, B3) and reduction rolls (R1, R2, R3), and each roll is connected to a sieving process per line, and six flour fractions derived from by-products (bran, short) and each fraction line (B1, R1, B2, R2, B3, R3) were finally produced. The dry milling procedure using the 'Buhler MLU-202' was carried out according to the reference literature (Methods 26-10, 26-20, 26-21A, 26-30A, 26-31, 26-41, Approved Methods of the American Association of Cereal Chemists, 10th Edition. 2000. St. Paul, USA). In the present invention, rice flour produced by dry milling was evaluated by summing all rice flour produced from the six fractions.

### Example 7-3: Analysis of Grain Hardness and Physicochemical Properties of Rice Flour

A laser diffraction particle size analyzer (model LS 13 320, Beckman Clter, Inc.) was used to measure the mean particle size and particle size distribution (diameter distribution of flour) of rice flour produced by dry milling under the same conditions.

As a result, as shown in Table 7 below, it was confirmed that the mean particular size of the rice flour of 'Samkwang (SA)-flo3' (65.3 µm) was slightly larger than that of the previously developed 'Garumi 2' (61.5 µm) and was significantly smaller than that of the original cultivar 'Samgwang' (91.1 µm).

This tendency was well maintained in the mean particle size per fraction according to the size of rice flour, and it was reconfirmed that 'Samkwang(SA)-flo3' can be pulverized more finely than other rice cultivars only with dry milling at a level similar to the previously developed 'Garumi 2'

**[Table 7]**

| Comparison of Size Distribution of Rice Flour produced by Test Mill | | | | | | |
|---|---|---|---|---|---|---|
| Variety | Mean Particle Size (µm) | Mean particular size per fraction according to flour size (µm) | | | | |
| | | Bottom < 10% | Bottom <25% | Bottom < 50% | Bottom <75% | Bottom <90% |
| Samkwang (SA)-flo3 | 65.3±0.86 | 10.1±0.09 | 23.1±0.44 | 62.7±0.72 | 98.1±1.16 | 127.1±1.62 |
| 'Samkwang' | 91.1±0.73 | 27.7±0.58 | 58.7±0.73 | 91.5±0.88 | 122.8±0.97 | 150.7±1.09 |
| 'Garumi 2' | 61.5±1.36 | 10.7±0.10 | 22.7±0.41 | 56.3±1.08 | 91.5±2.25 | 122.2±3.03 |

### Example 7-4: Measurement of Damaged Starch Ratio of Rice Flour

When producing rice flour by dry milling using normal non-glutinous rice, the biggest concern is the physical damage of starch granules due to high grain hardness of rice. The damage to starch granules greatly affects dough properties. In addition, damaged starch granules are quickly hydrated and easily hydrolyzed by alpha- or beta-amylase and converted into fermentable sugar, which promotes excessive fermentation and reduces the volume of processed products due to the generation of inappropriate gas during processing. (Starch Damage in Advances in Cereal Science and Technology. Vol. VII. pp321-349, American Association of Cereal Chemists Inc. St. Paul, USA).

Accordingly, a commercial analysis kit (Starch Damage Assay Kit, Megazyme, Ireland) was used to evaluate the damaged starch ratio of rice flour produced by dry milling under the same conditions. In the kit, the milled rice flour was added to an enzyme reaction solution for reaction, and then color was developed to analyze the amount of damaged starch granules using a spectrophotometer.

As a result, as shown in FIG. 8, the damaged starch ratio in the rice flour of 'Samkwang(SA)-flo3' (6.0%) was not significantly different from that of 'Garumi 2' (4.9%), but was significantly lower than that of the original non-glutinous rice cultivar 'Samkwang' (12.0%).

Based on the above results, the 'Samkwang(SA)-flo3' of the present invention has floury endosperm properties almost equivalent to that of 'Garumi 2', and thus has been confirmed to be a rice cultivar suitable for producing high-quality rice flour with fine particles and low damaged starch content only by dry milling.

### Example 7-5: Evaluation of Other Physicochemical Properties of Rice Flour

As a means for identifying the phenotype of the rice flour of 'Samkwang(SA)-flo3' of the present invention produced using a wheat mill, the lightness of rice flour (CIE value) was measured under specific moisture content conditions, and the ash content of rice flour was also measured. The investigation and measurement methods used were carried out according to the reference literature (Approved Methods of the American Association of Cereal Chemists, 10th Edition. 2000. St. Paul, USA).

As can be seen in Table 6, the whiteness of the rice flour of 'Samkwang (SA)-flo3' of the present invention was, the ash content, which is generally known to have high positive correlation with the mixing rate of bran layer in the milling process, was 0.67 for 'Samkwang', 0.64 for 'Samkwang(SA)-flo3', and 0.58 for 'Garumi 2', and there was no significant difference between these rice flour.

From the foregoing, a skilled person in the art to which the present invention pertains will be able to understand that the present invention may be embodied in other specific forms without modifying the technical concepts or essential characteristics of the present application. In this regard, the exemplary embodiments disclosed herein are only for illustrative purposes and should not be construed as limiting the scope of the present invention. On the contrary, the present invention is intended to cover not only the exemplary embodiments but also various alternatives, modifications, equivalents, and other embodiments that may be included within the spirit and scope of the present invention as defined by the appended claims.

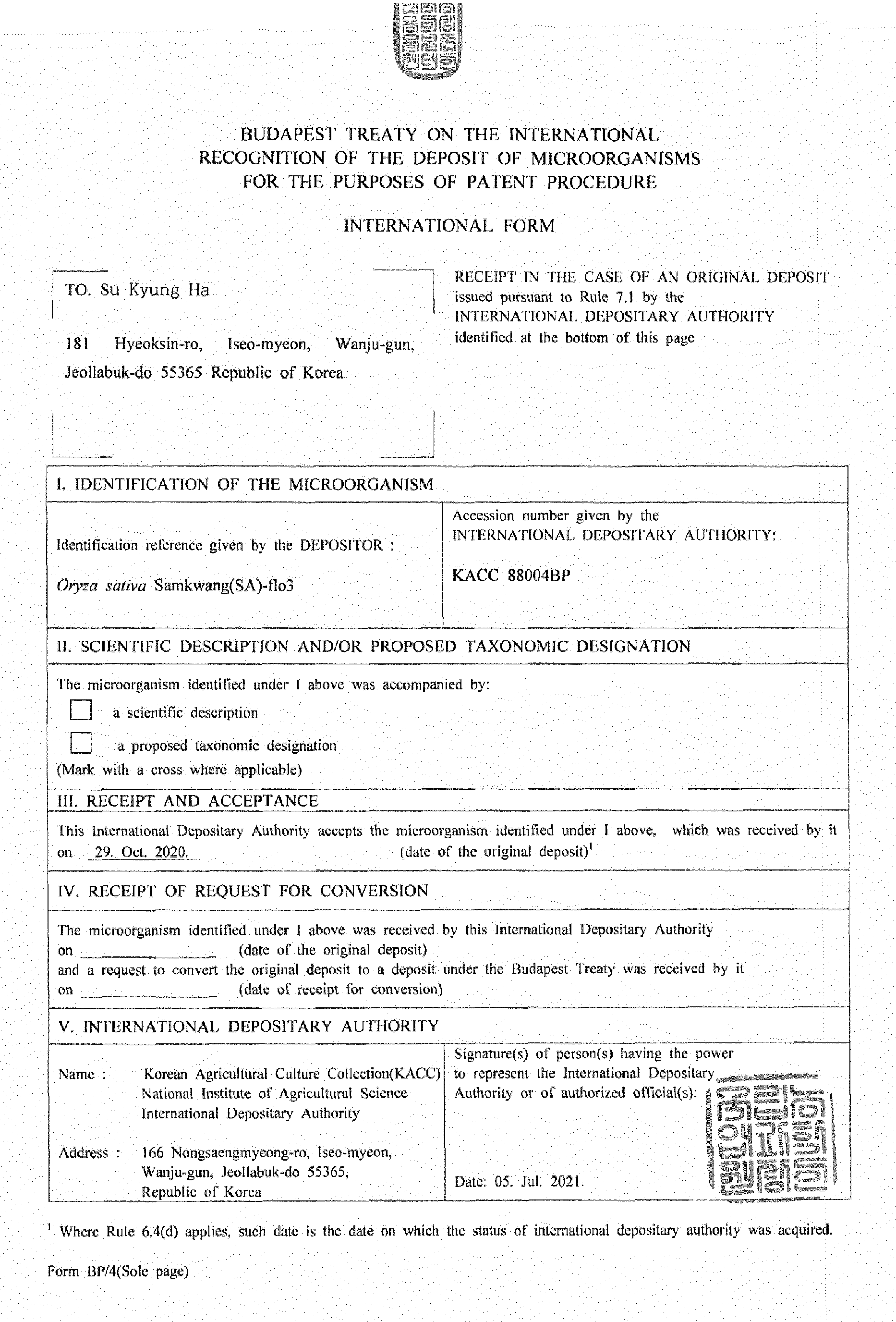

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

TO. Su Kyung Ha

181 Hyeoksin-ro, Iseo-myeon, Wanju-gun,
Jeollabuk-do 55365 Republic of Korea

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the DEPOSITOR :

Oryza sativa Samkwang(SA)-flo3 | Accession number given by the
INTERNATIONAL DEPOSITARY AUTHORITY:

KACC 88004BP
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above, which was received by it
on 29. Oct. 2020. (date of the original deposit)¹
IV. RECEIPT OF REQUEST FOR CONVERSION
The microorganism identified under I above was received by this International Depositary Authority
on ________________ (date of the original deposit)
and a request to convert the original deposit to a deposit under the Budapest Treaty was received by it
on ________________ (date of receipt for conversion)
V. INTERNATIONAL DEPOSITARY AUTHORITY
Name : Korean Agricultural Culture Collection(KACC)
National Institute of Agricultural Science
International Depositary Authority

Address : 166 Nongsaengmyeong-ro, Iseo-myeon,
Wanju-gun, Jeollabuk-do 55365,
Republic of Korea | Signature(s) of person(s) having the power
to represent the International Depositary
Authority or of authorized official(s):

Date: 05. Jul. 2021.

¹ Where Rule 6.4(d) applies, such date is the date on which the status of international depositary authority was acquired.

Form BP/4(Sole page)

## Claims

1. A 'Samkwang(SA)-flo3' rice cultivar, a mutant line derived from 'Samkwang' with floury endosperm characteristics, deposited under accession number KACC 98101P.

2. The rice cultivar according to claim 1, wherein the rice cultivar has the following characteristics:
(a) Heading date: August 14^{th} ±10 days
(b) Culm length (cm): 76±20
(c) Panicle length (cm): 19±10
(d) Tiller number (number): 15±5
(e) 1000-grain weight of brown rice (g): 18.5±5.0
(f) Length of brown rice (mm): 5.09±3.00
(g) Width of brown rice (mm): 2.97±2.00
(h) Length to width ratio of brown rice: 1.72±0.50.

3. A method for breeding 'Samkwang(SA)-flo3', a rice cultivar mutant line from 'Samkwang', comprising:
(a) immersing 'Samkwang' seeds in a sodium azide (NaN₃) solution, and advancing M2 seeds harvested from plants (M1) grown by germination thereof to M7 generation based on a line breeding method, thereby establishing fixed mutant lines; and
(b) selecting a rice cultivar with floury endosperm among the seeds of each established line.

4. The breeding method of claim 3, wherein the concentration of the diluted sodium azide solution in step (a) is 10 mM to 200 mM.

5. A composition for determining floury endosperm characteristics of rice in the next generation of 'Samkwang(SA)-flo3', comprising an agent capable of detecting a single nucleotide polymorphism (SNP) marker at a position corresponding to 19,721,940 bp of chromosome 5 in the standard genome (IRGSP 1.0) of rice.

6. The composition of claim 5, wherein the base of the SNP marker at the position corresponding to 19,721,940 bp of chromosome 5 in the standard genome (IRGSP 1.0) of rice is A.

7. The composition of claim 5, wherein the agent capable of detecting the SNP marker is a primer capable of amplifying a polynucleotide consisting of 10 to 300 bases containing a single nucleotide polymorphism site present at position 3,403 of the polynucleotide sequence represented by SEQ ID NO: 1, or a probe that specifically hybridizes with the polynucleotide.

8. The composition of claim 7, wherein the agent capable of detecting the SNP marker is a primer set consisting of primers represented by SEQ ID NO: 4 to SEQ ID NO: 7.

9. A kit for determining floury endosperm characteristics of rice in the next generation of 'Samkwang(SA)-flo3', comprising the composition of any one of claims 5 to 8.

10. A method for determining floury endosperm characteristics of rice in the next generation of 'Samkwang(SA)-flo3', comprising: detecting a SNP marker at a position corresponding to 19,725,941 bp of chromosome 5 in the standard genome (IRGSP 1.0) of rice.

11. The method of claim 10, wherein the base of the SNP marker at the position corresponding to 19,721,940 bp of chromosome 5 in the standard genome (IRGSP 1.0) of rice is A.

12. The method of claim 10, wherein the step of detecting a SNP marker comprises:
(a) performing PCR using a primer set consisting of primers represented by SEQ ID NO: 4 to SEQ ID NO: 7 based on the genomic DNA isolated from a sample as a template; and
(b) confirming the product amplified by the PCR.

13. A food composition comprising the rice cultivar of any one of claims 1 and 2, seeds thereof or extracts thereof, as an active ingredient.

14. The food composition of claim 13, wherein the food comprises rice flour produced from seeds of the rice cultivar.

15. The food composition of claim 14, wherein the rice flour is produced by dry milling.
